# EUROPEAN PATENT SPECIFICATION

(11) **EP 3 706 733 B1**
(45) Date of publication and mention of the grant of the patent: **27.04.2022**
(21) Application number: 18808500.5
(22) Date of filing: 05.11.2018
(51) Int. Cl.: A61K 31/352, A61K 45/06, A61P 15/00, A61K 36/48

(54) **SUBSTANCES AND COMPOSITIONS FOR THE USE IN THE TREATMENT OF ENDOMETRIOSIS AND ENDOMETRIOSIS ASSOCIATED SYMPTOMS**
SUBSTANZEN UND ZUSAMMENSETZUNGEN ZUR VERWENDUNG BEI DER BEHANDLUNG VON ENDOMETRIOSE UND ENDOMETRIOSE-ASSOZIIERTEN SYMPTOMEN
SUBSTANCES ET COMPOSITIONS DESTINÉES À ÊTRE UTILISÉES DANS LE TRAITEMENT DE L'ENDOMÉTRIOSE ET DE SYMPTÔMES ASSOCIÉS À L'ENDOMÉTRIOSE

(30) Priority: 09.11.2017 IT 201700127840
(43) Date of publication of application: 16.09.2020
(73) Proprietor: Sistemi Salute S.r.l., 80121 Napoli (IT)
(72) Inventor: SIGNORILE, Pietro Giulio, 00189 Roma (IT)
(74) Representative: Di Giovine, Paolo
(86) International application number: PCT/IB2018/058667
(87) International publication number: WO 2019/092579

(56) References cited:
- DATABASE WPI Week 201008 Thomson Scientific, London, GB; AN 2009-R45808 XP002782362, & CN 101 574 338 A (SHANGHAI INST PHARM IND) 11 November 2009 (2009-11-11)
- DATABASE WPI Week 201365 Thomson Scientific, London, GB; AN 2013-P13249 XP002782363, & CN 103 007 234 A (QINGDAO HUAREN INFORMATION TECHNOLOGY) 3 April 2013 (2013-04-03)
- KÜPELI AKKOL ESRA ET AL: "Phytochemical analyses and effects ofAlchemilla mollis (Buser) Rothm. and Alchemilla persica Rothm. in rat endometriosis model", ARCHIVES OF GYNECOLOGY AND OBSTETRICS, SPRINGER VERLAG, BERLIN, DE, vol. 292, no. 3, 21 February 2015 (2015-02-21), pages 619-628, XP035527740, ISSN: 0932-0067, DOI: 10.1007/S00404-015-3665-6 [retrieved on 2015-02-21]
- SÉRGIO REIS SOARES ET AL: "Pharmacologic therapies in endometriosis: a systematic review", FERTILITY AND STERILITY., vol. 98, no. 3, 1 September 2012 (2012-09-01), pages 529-555, XP055486800, USA ISSN: 0015-0282, DOI: 10.1016/j.fertnstert.2012.07.1120

## Description

### FIELD OF THE INVENTION

The present invention relates to Luteolin 2-(3,4-dihydroxyphenyl)-,5,7dihydroxy-4-chromenone for use a method for the treatment of endometriosis and symptoms thereof. The present invention further relates to compositions comprising Luteolin for use according to any administration route in the treatment of endometriosis, alone or associated to other substances, such as for example antiandrogens, antiprogestins, progestins and other flavonoids.

### STATE OF ART

Endometriosis, a disease affecting more than 150,000,000 of women in the Western world is a disease with chronic development therefor currently there are no medical therapies apt to reduce it or to remove the disease.

Endometriosis is characterized by invalidating symptoms affecting young women with serious invalidating physical discomforts, difficulties in the relationship life and infertility. Such symptoms are due to the inflammatory process determined by the development of disease outbreaks more frequently affecting pelvic organs, genitals, intestine and bladder, apart from their supporting structures. The factors which are altered in the acute and chronical inflammatory processes are several, a reduction in such factors reduces the inflammatory process which in endometriosis is responsible for the symptoms (acute and chronic pelvic pain, dyspareunia, dysmenorrhea, pain related to ovulation, pain due to urination, pain in the intestinal functions, chronic physical fatigue, headache, female infertility).

It is known that a decrease in estrogens favours a smaller disease activity with a lower immune response of the host which translates into lower acute and chronical inflammation. The factors involved in this inflammatory process are prostaglandins PGE 2, TNF alfa, metalloproteinases, VEGF. Estrogens, by increasing in vivo activity of the disease outbreaks, increase the inflammatory response of the affected organs.

CN 101 574 338 A discloses pharmaceutical compositions comprising a flavone (e.g. quercetin, kaempferol, isorhamnetin and apigenin) for reducing aromatase activity, useful for treating or preventing estrogen dependent diseases, including *inter alia* endometriosis. CN 103 007 234 A discloses a traditional Chinese medicinal preparation for treating dysmenorrhea caused by endometriosis, comprising a mixture of 21 plants, including extracts of perilla, dandelion and salvia.

The object of the present invention is to provide a new substance and composition for use in the treatment of endometriosis in all forms thereof and in any parts of the body. .

### SUMMARY OF THE INVENTION

The present invention is based on the finding that Luteolin is effective in the treatment of endometriosis. From data resulted from the herein reported clinical trial the effectiveness and the usefulness of Luteolin in the treatment of endometriosis is demonstrated. In particular, the treatment with Luteolin has demonstrated that in the group of patients affected by endometriosis with serious IV stage with "*deep endometriosis*", adenomyosis and ovarian disease, the treatment with Luteolin determines a quick reduction in the disease parameters with a net reduction in the "deep *endometriosis",* adenomyosis and ovarian endometriosis, apart from a quick regression in symptoms which almost disappeared already after only five days from the beginning of administration. The treated patients declared immediately their net well-being and with enthusiasm. Luteolin further reduced the values of the specific marker ca 125. An additional advantage highlighted by experimentation is that the beneficial effect is obtained with relatively low dosages of Luteolin. Moreover, neither side effects nor voluntary suspensions of the treatment by patients due to the optimum *compliance* of therapy were found.

The present invention thus relates to Luteolin and to compositions comprising Luteolin for use in the treatment of endometriosis.

Other advantages and features of the present invention will result evident from the following detailed description.

### DETAILED DESCRIPTION OF THE INVENTION

The present invention relates to Luteolin and to compositions including it for use in a method for the treatment of endometriosis.

Luteolin is a flavonoid with the structure formula shown hereinafter:

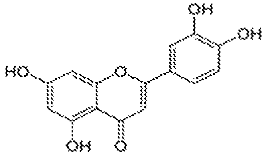

This substance is present in different plants, often it is present in the leaves, but it is also present in peels, bark, Trifolium flower and Ambrosia pollen. It was further insulated from aromatic plants, for example *Tomato salvia,* mint, family of *Lamiaceae.* It is also present in plants which are part of the diet such as celery, broccoli, peppers, parsley, thyme, dandelion, perilla, camomile tea, carrots, olive oil, peppermint, rosemary, in *Aiphanes aculeata* palm seeds, shells of peanuts. The compositions according to the present invention could include Luteolin purified from the above-described extracts or the extracts themselves, preferably titrated for their content of Luteolin. The herein described extracts could be obtained with the conventional techniques or purchased as commercially available.

The compositions preferably will have a concentration of Luteolin per dosage unit to be administered between 0.01 mg and 100 grams, more preferably between 10 and 500 mg, still more preferably between 50 and 200 mg, in particular between 50 and 100 mg.

According to an embodiment the compositions will include apart from Luteolin one or more active ingredients useful in the treatment of endometriosis such as for example antiandrogens, antiprogestins, progestins and other flavonoids.

According to an embodiment the compositions could further include one or more of the following compounds quercetin, curcuma extract or curcuma, feverfew extract and a preparation comprising omega-3 and/or omega-6, nicotinamide and/or 5-methyltetrahydrofolate calcium salt.

In the present description under the term preparation comprising omega-3 and/or omega-6 any preparation is meant, for example of natural, animal or plant origin, comprising the essential fatty acids omega-3 and/or omega-6, preferably both of them. According to an embodiment the composition will comprise a concentration of omega-3 between 200 and 3000 mg and/or of omega-6 between 50 and 2000 mg, in particular per dosage unit to be administered. Examples of omega-3- and omega-6-based preparations are linseed oil, fish oil, chia seeds, rapeseed oil. Linseed oil can be obtained by the person skilled in the art by means of conventional extraction techniques, for example it is obtained by squeezing previously dried or toasted linseeds, preferably the linseed oil in the composition will be in concentration between 0.3 and 7 grams, in particular per dosage unit to be administered. According to an embodiment Linseed oil will be used (Linum usitatissimum L., seeds, carrier: silicon dioxide) plv., Coating agent: Hydroxypropylmethycellulose; Charging agent: Dibasic calcium phosphate). According to an embodiment the linseed oil used in the composition is titrated in linoleic acid and/or α-linolenic acid, for example α-linolenic acid between 400 mg and 2000 mg.

The composition of the invention could include even a plant extract belonging to the curcuma genus, in particular belonging to the *curcuma longa* species. For example, in the composition there can be a curcuma extract in a dose between 15 e 200 mg, preferably 20 mg, such extract will include the active principle curcuma titrated for example in the above-said range. Curcuma determines a decrease in the amount of estrogens, inhibition of metalloproteinases, it speeds-up cellular apoptosis, it decreases TNF alfa immunity mediator, it decreases the interleukin mediators, it inhibits angiogenesis by reducing VEGEF.

The composition of the invention could also include a plant extract belonging to the *Tanacetum parthenium (feverfew)* species. According to an embodiment in the composition and extract of titrated feverfew could be present, for example in a dose between 3 and 100 mg, preferably 19.5 mg per dosage unit. Such extract will include the active principle parthenolide titrated for example in the above-mentioned range. Feverfew inhibits prostaglandins PGE(2), and TNF alfa, it inhibits the proliferation of fibroblasts, it has an anti-headache action and an action for reducing the inflammatory process. According to a preferred embodiment an extract of flower of feverfew titrated in parthenolide between 0.3 and 0.6% will be used, such as for example the one commercially available from LCM IPA num. STP-QCPJHS404-001.

The herein described extracts could be used with the conventional techniques or purchased as commercially available.

The composition of the invention could include even quercetin preferably in a dose between 50 mg and 1000 mg, in particular per dosage unit to be administered, preferably 200 mg. Quercetin decreases the local estrogens by reducing FSH and LH (studies on animal model), it reduces VEGEF. Quercetin and the obtaining/synthesis modes thereof are widely described in the state of known art and therefore they do not herein require additional investigations, by way of example it can be extracted from hawthorn, Marigold, horse chestnut, in particular Quercetin from flowers of *Sophora japonica* could be used. The composition of the invention could further include nicotinamide and/or 5-methyltetrahydrofolate calcium salt. 5-methyltetrahydrate calcium salt decreases homocysteine (thromboembolic factor), whereas Nicotinamide (Vitamin B3) carries out an antiangiogenetic action. According to an embodiment in the composition Nicotinamide can be present, for example in a dose between 5 and 100 mg, preferably 20 mg, whereas 5-methyltetrahydrofolate calcium salt preferably in a dose between 100 mg and 600 mg. The compositions according to the present invention can be formulated under any form and administration route and associated to any other component, in a variety of ways for example capsules, soft capsules, tablets, pills, jelly, powders or granules. Such excipients can be selected for example among those usually known in the state of art and include, but they are not limited thereto: a) carriers, such as for example sodium citrate and calcium phosphate, b) fillers such as for example starch, lactose, microcrystalline cellulose, sucrose, glucose, mannitol and colloidal silica, c) wetting agents, such as for example glycerol, d) disintegrating agents, such as alginates, calcium carbonate, starches, derivatives of starch, of cellulose and of polyvinylpyrrolidone, silicates and sodium carbonate e) binders such as carboxymethylcellulose, alginates, jelly, polyvinylpyrrolidone, sucrose, cellulose polymeric derivatives, starch derivatives f) retardants such as paraffin, cellulose polymers, esters of fatty acids g) absorption accelerators, such as quaternary ammonium compounds, h) wetting agents and surfactants such as cetyl alcohol and glycerol monostearate, i) adsorbents, such as benthic clays and kaolin, k) lubricants such as talcum, calcium stearate, magnesium stearate, polyethylene glycol, sodium lauryl sulphate, sodium stearyl j) glidants such as talcum, colloidal silica.

The forms of solid dosage, such as tablets, capsules, soft capsules, jelly, pills and granules could be coated with enteric, gastric coatings or coatings of other type known in the state of art. They can include opacifiers and they can be of the type as to allow the release of the active ingredients only or preferably in a determined tract of the intestine, in case in delayed way. Substances which can allow such delayed use include, but they are not limited thereto, polymers and waxes.

The soft capsules could house the active antioxidant substances in liquid form alone or in solutions, suspensions or emulsions of the active substances in a liquid solvent. The soft capsules could be characterized by a casing qualitatively similar to that of the stiff capsules, but thicker and softer.

Liquid forms suitable to an oral administration for example are emulsions, solutions, prepared or extemporaneous suspensions, syrups and elixirs. Excipients suitable to the formulations according to the present invention in liquid forms for oral use include, but they are not limited thereto, diluents such as water or other solvents, solubilizing agents and emulsifiers selected among ethyl alcohol, polyhydric alcohols, propylene glycol, glycerol, polyethylenglycol and sorbitan esters. These formulations can even include sweeteners and flavours.

The compositions will be for example a food supplement, a nutraceutical composition, a dietary and nutritional food product, a food product, a beverage, a medical device, a nutraceutical, a medicament, a medicated food, a food for special medical purposes, food, a pharmaceutical composition. The compositions will be mainly intended to be used by the human beings, but they could be used even on animals.

The compositions according to the present invention could include one or more suitable food ingredients and/or preservatives and/or acidifiers and/or antioxidants and/or immuno-stimulating agents and/or dyes and/or probiotics and/or prebiotics.

The combination of the above-mentioned active ingredients could be used formulated in one single composition according to the several above-described embodiments or in a kit including the different separated ingredients, for example in single compositions such as capsules, pills, tablets for sequential or contemporary administration of the different ingredients.

The above-described compositions could be used/administered/ingested for the treatment of endometriosis, in particular for the treatment of the symptoms associated to this pathology such as for example acute and chronic pelvic pain, dyspareunia, dysmenorrhea, pain related to ovulation, pain due to urination, pain in the intestinal functions, chronic physical fatigue, headache, female infertility. Preferably, independently from the used type of formulation, Luteolin according to the present invention will be administered with a daily dosage regime between 30 and 200 mg/die preferably between 100 and 150 mg/die.

### EXPERIMENTAL SECTION

### Clinical study on patients affected by endometriosis treated with Luteolin

A composition in powder for oral administration containing Luteolin (62.5 mg of extract of shells of peanuts standardized to 80% Luteolin) equal to 50 mg was prepared.

18 patients affected by endometriosis, IV stage, were identified, all of them with *deep endometriosis,* adenomyosis and ovarian endometriosis (endometriosis ovarian cysts larger than 3 cm), the disease diagnosis was placed with diagnostic protocol including vaginal and rectal explorations for the diagnosis of deep endometriosis, Nuclear magnetic resonance of pelvis with examination of resonance parameters for the diagnosis of adenomyosis, thickness of the neck-body junction area (JZ) of the uterus larger than 12 mm, exclusion criterion for adenomyosis thickness smaller than 8 mm, examination of the myometrium structure, examination of the myometrium thicknesses both of front and rear wall, hematic examinations comprising the marker Ca 125, the dosage of 17B-estradiol and of testosterone.

The first group constituted by 18 patients ingested 2 capsules per day, every twelve hours, for 45 days of the composition containing Luteolin 50 mg.

For the disease evaluation:
The dosage of Ca 125
The dosage of 17B-estradiol
The dosage of testosterone
The Rectal examination for deep endometriosis
The rectal examination for deep endometriosis
The nuclear magnetic resonance of pelvis with contrast medium (gadolinium)
were performed.

Average age of the affected study group treated with Luteolin: 36.3 years.
INCLUSION CRITERIA for experimentation
Regular menstrual period
Not ingestion of pain-relieving drugs
Absence of anti-inflammatory drugs
Absence of hormone drugs
Positive Vaginal examination
Positive rectal examination
Ca125 higher than 40
VAS scale higher than 7
Group features
Headache: 21%
Cystitis: 23%
Dysmenorrhea VAS >= 7
Dyspareunia VAS>= 7
Chronic pelvic pain VAS>= at 7
Basal serum examinations
Ca 125 dosage: average value = 72.6 UI/ml
17 Beta-estradiol dosage (21^{st} day of the period)
basal average value of the sample: 199 pg/ml
Serum testosterone dosage (21^{st} day of the period)
basal average value of the sample: 0.13 ng/ml
RMN Adenomyosis JZ > at 12 mm
RMN ovarian cyst sizes (maximum diameter) average weighted on 18 cases = cm 5.34
Study group 21^{st} day of treatment
Headache: 11%
Cystitis: 5%
Dysmenorrhea VAS <3= 100
Dyspareunia VAS<= 3=100
Chronic pelvic pain VAS<3=100
Serum examinations
Ca 125 dosage: average value = 32.1 UI/ml
17 Beta-Estradiol dosage (21^{st} day of period) average value of the sample: 196 pg/ml
Serum dosage of testosterone average value of the sample 0.15 ng/ml
RMN Adenomyosis JZ < 8 mm 95%, between 12 and 8mm equal to 5%
RMN ovarian cysts after 21 days of weighted average therapy 2.14

The treatment with Luteolin by oral route twice a day, every 12 hours with dosage for single administration of 50mg, for a daily total of 100mg, soon demonstrated in the group of 18 patients affected by endometriosis, serious IV stage, with *deep endometriosis,* adenomyosis and ovarian disease a quick decrease in the disease parameters with a net decrease in the deep endometriosis, of adenomyosis, JZ < at 12mm and of ovarian endometriosis, average maximum diameter of the cysts from cm 5.34 to cm 2.14, apart from a quick regression of the symptoms which almost disappeared already after five days as from the beginning of administration, such result proceeded constantly for the whole duration of administration (45 days). After the administration of Luteolin 50mg twice a day, a negativization of antigen marker Ca 125 in all treated patients occurred. No side effects nor voluntary suspensions of the treatment by the patients for the optimum *compliance* of the therapy were found. The treated patients declared immediately their net well-being and with enthusiasm.

## Claims

1. Luteolin for use in the treatment of endometriosis.

2. A composition for use in the treatment of endometriosis comprising Luteolin and optionally one or more excipients and/or diluents and/or carriers.

3. The composition for use according to claim 2 further comprising one or more antiandrogens, antiprogestins, progestins, other flavonoids.

4. The composition for use according to claim 2 or 3 wherein said Luteolin per dosage unit is between 0.01 mg and 100 grams, preferably between 10 and 500 mg, still more preferably between 30 and 200 mg.

5. The composition for use according to any one of claims 2 to 4 in the treatment of one or more symptoms associated with endometriosis selected among acute and chronic pelvic pain, dyspareunia, dysmenorrhea, pain related to ovulation, pain due to urination, pain in the intestinal functions, chronic physical fatigue, headache, female infertility and decrease in endometriosis outbreaks.

6. The composition for use according to any one of claims 2 to 5 comprising a plant extract containing said Luteolin.

7. The composition for use according to claim 6 wherein said plant extract is an extract of Clover, Ambrosia pollen, *Tomato salvia,* mint, celery, broccoli, peppers, parsley, thyme, dandelion, perilla, camomile tea, carrots, olive oil, peppermint, rosemary, *Aiphanes aculeata* palm seeds, shells of peanuts.

8. The composition according to any one of claims 2 to 7 further comprising one or more of the following substances quercetin, an extract of a plant belonging to the genus curcuma or curcumin, an extract of a plant belonging to the species feverfew (*Tanacetum parthenium),* a preparation comprising omega-3 and/or omega-6, nicotinamide and/or 5-methyltetrahydrofolate calcium salt, linseed oil.

9. The composition for oral use according to any one of claims 1 to 8 in a form selected from capsule, soft capsule, tablet, pill, jelly, powder, granule, emulsion, solution, syrup, suspension.

10. The composition according to any one of claims 1 to 9 wherein said composition is a food supplement, a nutraceutical composition, a dietary and nutritional food product, a food product, a beverage, a nutraceutical, a medicament, a medicated food or a food for special medical purposes, a medical device, a pharmaceutical composition.

## Patentansprüche

1. Luteolin zur Verwendung bei der Behandlung von Endometriose.

2. Stoffgemisch zur Verwendung bei der Behandlung von Endometriose umfassend Luteolin und gegebenenfalls einen oder mehrere Hilfsstoffe und/oder Verdünnungsmittel und/oder Träger.

3. Stoffgemisch zur Verwendung gemäß Anspruch 2 ferner umfassend ein oder mehrere Antiandrogene, Antiprogestine, Progestine, andere Flavonoide.

4. Stoffgemisch zur Verwendung gemäß Anspruch 2 oder 3, wobei das Luteolin pro Dosierungseinheit zwischen 0,01 mg und 100 Gramm, vorzugsweise zwischen 10 und 500 mg, noch bevorzugter zwischen 30 und 200 mg ist.

5. Stoffgemisch zu Verwendung gemäß einem der Ansprüche 2 bis 4 bei der Behandlung einer oder mehrerer mit Endometriose verbundener Symptome, ausgewählt unter akuten und chronischen Beckenschmerzen, Dyspareunie, Dysmenorrhoe, Schmerzen im Zusammenhang mit dem Eisprung, Schmerzen aufgrund von Wasserlassen, Schmerzen in den Darmfunktionen, chronischer körperlicher Müdigkeit, Kopfschmerzen, weibliche Unfruchtbarkeit und Mindern von Endometrioseausbrüchen.

6. Stoffgemisch zur Verwendung gemäß einem der Ansprüche 2 bis 5 umfassend einen Pflanzenextrakt beinhaltend das Luteolin.

7. Stoffgemisch zur Verwendung gemäß Anspruch 6, wobei der Pflanzenextrakt ein Extrakt aus Klee, Ambrosiapollen, *Tomato salvia*, Minze, Sellerie, Brokkoli, Paprika, Petersilie, Thymian, Löwenzahn, Perilla, Kamillentee, Karotten, Olivenöl, Pfefferminz, Rosmarin, *Aiphanes aculeata*-Palmensamen, Erdnussschalen ist.

8. Stoffgemisch gemäß einem der Ansprüche 2 bis 7 ferner umfassend eine oder mehrere der folgenden Substanzen: Quercetin, einen Extrakt einer Pflanze die der Gattung Curcuma oder Curcumin angehört, ein Extrakt einer Pflanze die der Gattung Mutterkraut (*Tanacetum parthenium*) angehört, eine Zubereitung umfassend Omega-3 und/oder Omega-6, Nicotinamid und/oder 5-Methyltetrahydrofolat-Kalziumsalz, Leinöl.

9. Stoffgemisch zur oralen Verwendung gemäß einem der Ansprüche 1 bis 8 in einer Form ausgewählt aus Kapsel, Weichkapsel, Tablette, Pille, Gelee, Puder, Granulat, Emulsion, Lösung, Sirup, Suspension.

10. Stoffgemisch gemäß einem der Ansprüche 1 bis 9, wobei das Stoffgemisch ein Nahrungsergänzungsmittel, ein nutrazeutisches Stoffgemisch, ein diätetisches und nahrhaftes Lebensmittelprodukt, ein Lebensmittelprodukt, ein Getränk, ein Nutrazeutikum, ein Medikament, ein medikamentiertes Lebensmittel oder ein Lebensmittel für besondere medizinische Zwecke, ein medizinisches Gerät oder ein pharmazeutisches Stoffgemisch ist.

## Revendications

1. Lutéoline pour une utilisation dans le traitement de l'endométriose.

2. Composition pour une utilisation dans le traitement de l'endométriose, comprenant de la lutéoline et éventuellement un ou plusieurs excipients et/ou diluants et/ou véhicules.

3. Composition pour une utilisation selon la revendication 2, comprenant en outre un ou plusieurs anti-androgènes, anti-progestines, progestines, autres flavonoïdes.

4. Composition pour une utilisation selon la revendication 2 ou 3, dans laquelle ladite lutéoline par unité posologique est comprise entre 0,01 mg et 100 grammes, de préférence entre 10 et 500 mg, mieux encore entre 30 et 200 mg.

5. Composition pour une utilisation selon l'une quelconque des revendications 2 à 4, dans le traitement d'un ou plusieurs symptômes associés à une endométriose choisis parmi une douleur pelvienne aiguë ou chronique, une dyspareunie, une dysménorrhée, une douleur liée à l'ovulation, une douleur due à la miction, une douleur dans les fonctions intestinales, une fatigue physique chronique, une céphalée, une stérilité féminine, et une diminution des poussées d'endométriose.

6. Composition pour une utilisation selon l'une quelconque des revendications 2 à 5, comprenant un extrait végétal contenant ladite lutéoline.

7. Composition pour une utilisation selon la revendication 6, dans laquelle ledit extrait végétal est un extrait de trèfle, de pollen d'ambroisie, de *Tomato salvia,* de menthe, de céleri, de brocoli, de poivron, de persil, de thym, de pissenlit, de perilla, de camomille pour infusion, de carotte, d'huile d'olive, de menthe poivrée, de romarin, de graines de palmier *Aiphanes aculeata,* de coques de cacahuètes.

8. Composition selon l'une quelconque des revendications 2 à 7, comprenant en outre une ou plusieurs des substances suivantes : la quercétine, un extrait d'une plante appartenant au genre curcuma ou la curcumine, un extrait d'une plante appartenant à l'espèce de la grande camomille (*Tanacetum parthenium*), une préparation comprenant des oméga-3 et/ou oméga-6, le nicotinamide et/ou le sel 5-méthyltétrahydrofolate de calcium, l'huile de lin.

9. Composition à usage oral selon l'une quelconque des revendications 1 à 8, sous une forme choisie parmi une capsule, une capsule molle, un comprimé, une pilule, une gelée, une poudre, un granule, une émulsion, une solution, un sirop, une suspension.

10. Composition selon l'une quelconque des revendications 1 à 9, laquelle composition est un complément alimentaire, une composition nutraceutique, un produit alimentaire diététique et nutritionnel, un produit alimentaire, une boisson, un nutraceutique, un médicament, un aliment médicamenteux ou un aliment à des fins médicales spécifiques, un dispositif médical, une composition pharmaceutique.
